# EUROPÄISCHE PATENTANMELDUNG

(11) **EP 2 045 410 A1**
(43) Veröffentlichungstag der Anmeldung: **08.04.2009**
(21) Anmeldenummer: 07117629.1
(22) Anmeldetag: 01.10.2007
(51) Int. Cl.: E04C 2/04, A61L 9/20, D21H 13/00

(54) **Bauplatte auf Basis von Gips**

(71) Anmelder: Knauf Gips KG, 97346 Iphofen (DE)
(72) Erfinder: Hummel, Hans-Ulrich, Prof., 97346 Iphofen (DE)
(74) Vertreter: Schreiber, Christoph

(57) **Zusammenfassung**

Bauplatte auf Basis von Gips mit einer Abdeckung aus Glasfaservlies auf beiden Hauptflächen, **dadurch gekennzeichnet, dass** das Glasfaservlies auf mindestens einer der beiden Hauptflächen eine Beschichtung aufweist, die optimiertes photokatalytisch aktives Titandioxid enthält.

## Beschreibung

Die vorliegende Erfindung betrifft eine Bauplatte auf Basis von Gips und Verfahren zu ihrer Herstellung.

Bauplatten auf Basis von Gips werden im großen Umfang insbesondere im Innenausbau von Gebäuden eingesetzt, insbesondere zur Herstellung von Decken und Wänden.

Die übliche Bauplatte auf Basis von Gips ist eine sogenannte Gipskartonplatte, bei der die Gipsmasse beidseitig mit einer Kartonschicht abgedeckt ist. Es sind verschiedene Spezialplatten bekannt, die beispielsweise statt einer Abdeckung mit einem Karton eine Abdeckung mit einem Vlies aufweisen. Solche sind beispielsweise in der EP 0 755 903 A1 beschrieben.

Die steigenden Anforderungen an die Wärmedämmung von Gebäuden führen dazu, dass die Gebäude annähernd "luftdicht" gegen die Außenwelt abgeschlossen sind und sich daher die Luftwechselraten in den Räumen stark reduziert haben. Hierdurch werden Belastungen durch Gerüche und Luftschadstoffe zu einem vermehrten Problem.

Die vorliegende Erfindung hat sich daher die Aufgabe gestellt, Materialien bereitzustellen, um die Belastungen von Innenräumen mit Luftschadstoffen und Gerüchen zu verringern.

Gelöst wird die Aufgabe durch eine Bauplatte auf Basis von Gips mit einer Abdeckung aus Glasfaservlies auf beiden Hauptflächen, dadurch gekennzeichnet, dass das Glasfaservlies auf mindestens einer der beiden Hauptflächen eine Beschichtung aufweist, die photokatalytisch aktives Titandioxid enthält.

Erfindungsgemäß handelt es sich um eine Bauplatte mit einer beidseitigen Glasfaservliesabdeckung, wobei zumindest eine Seite eine Beschichtung aufweist, die photokatalytisch aktives auf der Außenseite Titandioxid enthält.

Photokatalytische Materialien sind Halbleiter, bei denen unter Lichteinwirkung Elektronen-Loch-Paare entstehen, welche an der Oberfläche mit diversen Luftbestandteilen Redox-Reaktionen hervorrufen können.

Als photokatalytisch aktives Titandioxid wird bevorzugt ein Titandioxid eingesetzt, wie es in der EP 1 178 011 A1, EP 1 254 863 und insbesondere in der WO 2005/108505 beschrieben ist.

Typische Gehalte des photokatalytisch aktiven Titandioxids im Glasfaservlies betragen bis zu 25 Gew.-%.

Die Beschichtung des Glasfaservlieses muss zum einen eine gute Bindung an den Gipskern erlauben, auf der anderen Seite das photokatalytisch aktive Titandioxid fest binden aber nicht so einschließen, dass es dieses komplett und lichtundurchlässig umschließt. Das photokatalytisch aktive Titandioxid befindet sich bevorzugt als Coatingbestandteil auf der dem Gipskern abgewandten Seite der Bauplatte.

Eine besonders geeignet Zusammensetzung eines Coatings für ein Glasfaservlies weist folgende Zusammensetzung auf:
- 0 bis 30 Gew.-% Füllstoff wie Calciumsulfatanhydrit oder Kalksteinmehl
- 0 bis 6 Gew.-% organisches Bindemittel, insbesondere Polyvinylacetat
- 0 bis 90 Gew.-% Titandioxid (photokatalytisch aktiv)
- 0,01 bis 0,8 Gew.-% Celluloseether
- 0,01 bis 1,0 Gew.-% Verflüssiger, maximal 0,2 Gew.-% Netzmittel.

Zusätzlich kann das Produkt übliche Hilfsmittel wie Plastifizierer, Fungizide, etc. enthalten.

Als Verfahren zur Herstellung hat sich insbesondere ein Verfahren erwiesen, das folgende Schritte aufweist:
- ein Glasfaservlies mit einer Beschichtung auf der dem Gipskern abgewandten Seite versehen wird, die photokatalytisch aktives Titandioxid enthält,
- ein beschichtetes oder unbeschichtetes Glasfaservlies mit einem abbindefähigen Calciumsulfatgemisch beladen wird,
- anschließend mit einem beschichteten oder unbeschichteten Glasfaservlies abgedeckt wird,
- nach dem Beginn des Abbindens geschnitten und getrocknet wird;
wobei mindestens eine der Hauptflächen der Bauplatte ein beschichtetes Glasfaservlies aufweist.

Gegenstand der Erfindung ist auch ein Verfahren zur Entfernung von Luftschadstoffen oder Gerüchen aus Innenräume, umfassend den Schritt des Anbringens einer erfindungsgemäßen Bauplatte in einem Innenraum sowie die Verwendung der erfindungsgemäßen Bauplatte zur Verringerung von Luftschadstoffen und Gerüchen in Innenräumen.

## Patentansprüche

1. Bauplatte auf Basis von Gips mit einer Abdeckung aus Glasfaservlies auf beiden Hauptflächen, **dadurch gekennzeichnet, dass** das Glasfaservlies auf mindestens einer der beiden Hauptflächen eine Beschichtung aufweist, die optimiertes photokatalytisch aktives Titandioxid enthält.

2. Bauplatte nach Anspruch 1, **dadurch gekennzeichnet, dass** die Beschichtung bis 90 Gew.-% photokatalytisch aktives Titandioxid enthält.

3. Bauplatte nach Anspruch 1 oder 2, **dadurch gekennzeichnet, dass** die Beschichtung des Glasfaservlieses folgende weitere Bestandteile aufweist:
- 0 bis 30 Gew.-% eines inerten Füllstoffes wie Calciumsulfat-Anhydrit oder Kalksteinmehl,
- 0,01 bis 0,8 Gew.-% Celluloseether,
- 0,01 bis 1,0 Gew.-% Verflüssiger,
- bis 0,2 Gew.-% Netzmittel.

4. Bauplatte nach mindestens einem der Ansprüche 1 bis 3, **dadurch gekennzeichnet, dass** die Beschichtung ein Flächengewicht von 280 bis 330 g/m² hat.

5. Verfahren zur Herstellung einer Bauplatte nach mindestens einem der Ansprüche 1 bis 4, **dadurch gekennzeichnet, dass**
- ein Glasfaservlies mit einer Beschichtung versehen wird, die optimiertes photokatalytisch aktives Titandioxid enthält.
- ein beschichtetes oder unbeschichtetes Glasfaservlies mit einem abbindefähigen Calciumsulfatgemisch beladen wird
- anschließend mit einem beschichteten oder unbeschichteten Glasfaservlies abgedeckt wird
- nach dem Beginn des Abbindens geschnitten und getrocknet wird;
wobei mindestens eine der Hauptflächen der Bauplatte ein beschichtetes Glasfaservlies aufweist.

6. Verfahren zur Verringerung von Luftschadstoffen oder Gerüchen umfassend den Schritt des Anbringens einer Bauplatte nach mindestens einem der Ansprüche 1 bis 4 im Innenraum.

7. Verwendung einer Bauplatte nach mindestens einem der Ansprüche 1 bis 4 zur Verringerung von Luftschadstoffen und Gerüchen in Innenräumen.
